# EUROPEAN PATENT SPECIFICATION

(11) **EP 3 868 736 B1**
(45) Date of publication and mention of the grant of the patent: **30.11.2022**
(21) Application number: 20158781.3
(22) Date of filing: 21.02.2020
(51) Int. Cl.: C07C 37/50, C07C 67/30

(54) **SOLVENT-FREE METHOD FOR PREPARING CANNABINOIDS**
LÖSEMITTELFREIES VERFAHREN ZUR HERSTELLUNG VON CANNABINOIDEN
PROCÉDÉ SANS SOLVANT DE PRÉPARATION DE CANNABINOÏDES

(43) Date of publication of application: 25.08.2021
(73) Proprietor: SCI Pharmtech Inc, Taoyuan City 338 (TW)
(72) Inventor: WANG, Heng-Yen, 338 Taoyuan City (TW); LI, Feng-Hsu, 338 Taoyuan City (TW); YANG, Zhi-Jie, 338 Taoyuan City (TW); HUANG, Hsin-Yi, 338 Taoyuan City (TW)
(74) Representative: Icosa

(56) References cited:
- US-A1- 2004 033 280
- US-A1- 2017 349 517
- US-A1- 2017 349 547
- VERESS T ET AL: "Determination of cannabinoid acids by high-performance liquid chromatography of their neutral derivatives formed by thermal decarboxylation", JOURNAL OF CHROMATOGRAPHY A, ELSEVIER, AMSTERDAM, NL, vol. 520, 9 November 1990 (1990-11-09), pages 339-347, XP026743628, ISSN: 0021-9673, DOI: 10.1016/0021-9673(90)85118-F [retrieved on 1990-11-09]

## Description

### BACKGROUND

### 1. Technical Field

The present disclosure relates to methods for preparing cannabinoids, particularly to methods for preparing cannabidiol (CBD).

### 2. Description of Associated Art

Cannabidiol is a phytocannabinoid, one of the several cannabinoids identified in cannabis plants and accounts for up to 40% of the plant's extract. A method to extract pharmaceutically active components from plant materials for preparing botanical drug substances that comprises a heating step to decarboxylate the acid form of the cannabinoids (i.e., tetrahydrocannabinol acids and cannabidiol acids) to their neutral form (i.e., tetrahydrocannabinol and cannabidiol) has been disclosed in US 2004/033280 A1. For determining the cannabinoids in plant samples, due to instability of cannabidiol acids and tetrahydrocannabinol acids that tend to decompose into neutral cannabinoids easily, an indirect method is used to determine the cannabinoid acids by high-performance liquid chromatography of the neutral cannabinoids formed by thermal decarboxylation, as disclosed in an article by Veress T. et al. ("Determination of cannabinoid acids by high-performance liquid chromatography of their neutral derivatives formed by thermal decarboxylation," JOURNAL OF CHROMATOGRAPHY A, ELSEVIER, AMSTERDAM, NL, Vol. 520, 9 November 1990 (1990-11-09), pages 339-347, XP026743628, ISSN: 0021-9673, DOI: 10.1016/0021-9673(90)85118). A method for synthetically preparing crystalline cannabidivarin has been disclosed in US 2017/0349517 A1, comprising adding a solution of synthetically-prepared cannabidivarin in a suitable solvent to an antisolvent, and removing the suitable solvent to make crystalline cannabidivarin.

It has been demonstrated to afford synthetic cannabidiol (CBD) via hydrolysis-decarboxylation of its corresponding intermediate, terpene-substituted olivetolate. One of the early examples of synthetic cannabidiol preparation via such process has been reported by Petrzilka, W. et al. in Helvetica Chimica Acta 52, 4, pp. 1102-1134 (1969) and shown in Scheme 1 below. In Scheme 1, Me refers to a methyl group; Et refers to an ethyl group; and aq refers to an aqueous solution.

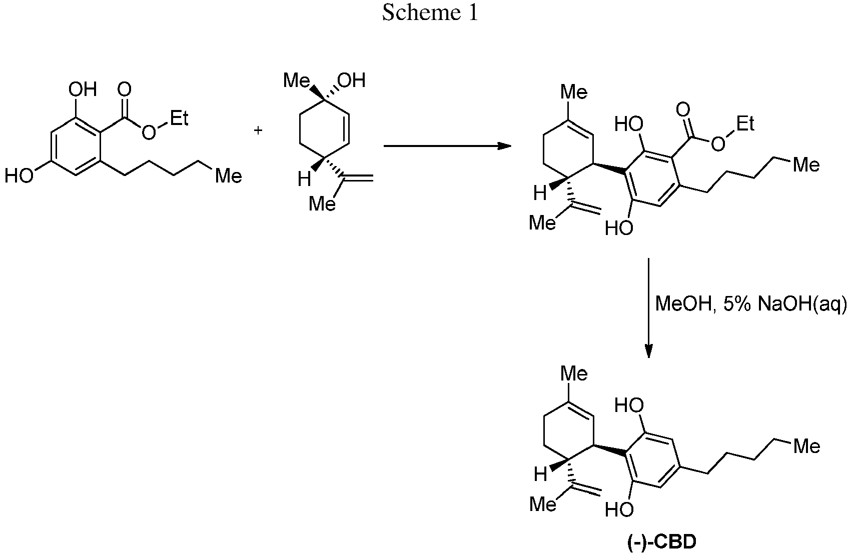

Another early example of cannabidiol synthesis by hydrolysis-decarboxylation of olivetolate has been described by Gaoni in Tetrahedron 21, 5, pp. 1223-1229 (1965) and illustrated in scheme 2 below.

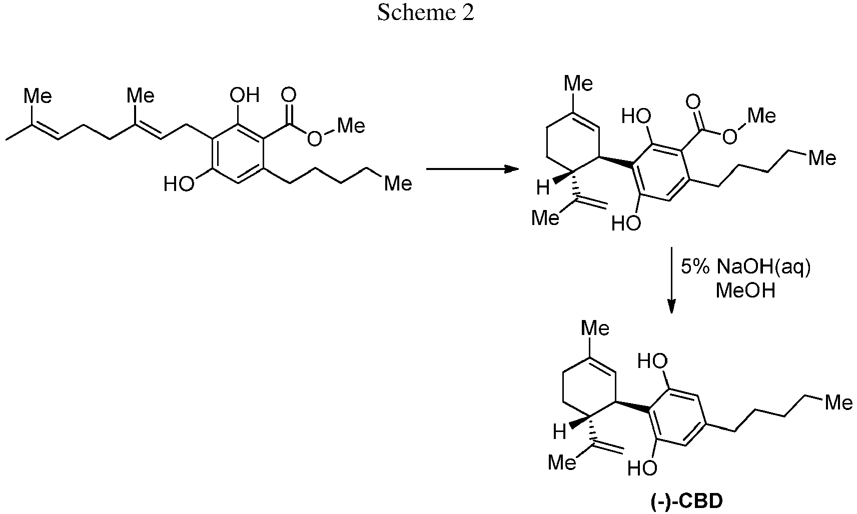

Recently, synthetic cannabidiol afforded through the process of hydrolysis-decarboxylation of cannabinoids was carried out with menthadienol-substituted ethyl olivetolate, as disclosed in U.S. Patent No. 7,674,922 (shown in scheme 3 below). In particular, the ethyl cannabidiolate was dissolved in methanol and added with a sodium hydroxide aqueous solution. The mixture was then heated to reflux and held for 3.5 hours, followed by cooling to room temperature. The reaction mixture was then quenched with aqueous citric acid. Thereafter, heptane was added to the mixture, and the product was extracted into the heptane phase. A second extraction using heptane was performed. The combined organics were dried by azeotropic distillation of the water and concentrated, followed by cooling to obtain solid cannabidiol. This synthesis method was able to achieve a 57.5% yield of cannabidiol over two steps from ethyl olivetolate.

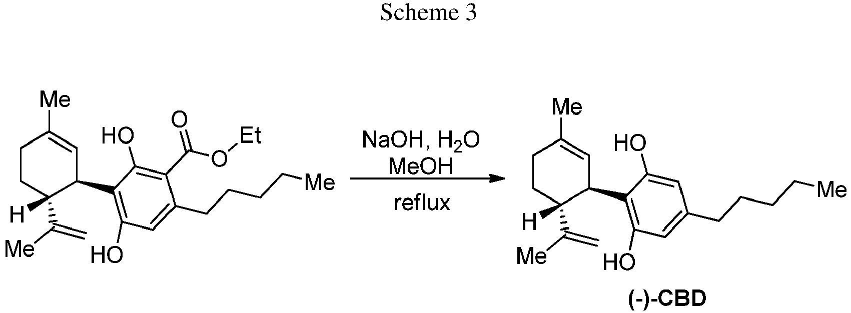

Moreover, European Patent No. 2,314,580 claimed to obtain a more than 95% reaction yield of hydrolysis-decarboxylation of menthadienol-substituted methyl olivetolate, by making use of a sealed reaction tank with a low boiling point solvent, such as MeOH (shown in scheme 4 below), or at atmosphere pressure with a high boiling point alcohol, such as ethylene glycol (shown in scheme 5 below).

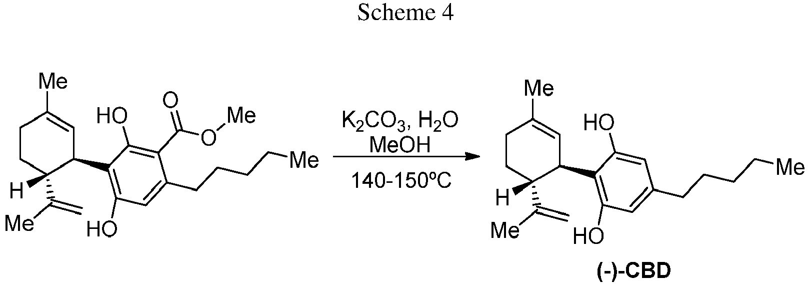

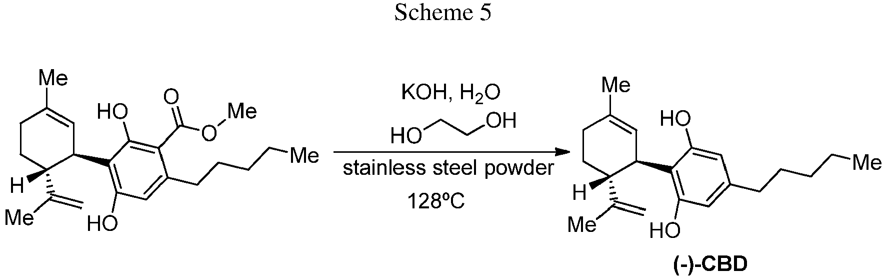

However, there were several concerns regarding the hydrolysis-decarboxylation method disclosed by European Patent No. 2,314,580 for industrial production. For example, the reaction was claimed to carry out in a low boiling point solvent under pressure and may pose significant safety hazard in bulk production. Specifically, combination of methanol and water was used as the solvent under the reaction temperature of 140 °C to 150 °C in a sealed reaction tank, which could produce a tremendous amount of methanol/water vapor in the sealed reaction tank and is hazardous in industrial scale production. In addition, the alternative method for the hydrolysis-decarboxylation reaction using high boiling point alcohol may require additional treatment to the aqueous organic waste after production. Further, using high boiling point alcohol may increase the risk of jeopardizing the quality of the cannabidiol product with a potential residual solvent, such as ethylene glycol, which is categorized as the Class II residual solvent in United States Pharmacopeia (USP) and should be avoided in pharmaceutical products.

Accordingly, there is still an unmet need in a safe, effective and environment-friendly method for the synthetic preparation of cannabidiol at industrial scale.

### SUMMARY

In view of the foregoing, the present disclosure provides a method for preparing cannabidiol represented by formula (I) below, 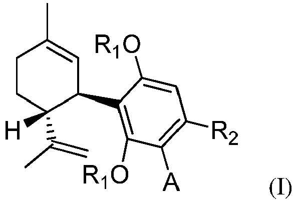 comprising hydrolysis-decarboxylation of a compound represented by formula (II) below, 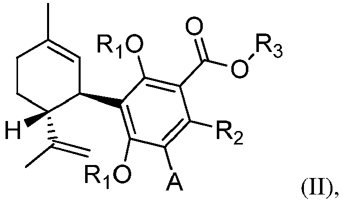 wherein R₁ is H, an alkyl group or a protecting group, R2 is an-alkyl group or an ether group, R3 is an alkyl group or a protecting group, and A is H or a carboxylic ester group.

### DETAILED DESCRIPTION OF THE EMBODIMENTS

The following illustrative embodiments are provided to illustrate the disclosure of the present specification. These and other aspects and effects can be understood by those skilled in the art after reading the present disclosure.

Prior to the present disclosure, alcohols, e.g., MeOH, are used as solvents in the hydrolysis-decarboxylation step of the cannabidiol synthesis. The present disclosure provides a method for preparing cannabidiol comprising hydrolysis-decarboxylation in an absence of a solvent. For example, the present disclosure provides a method for preparing cannabidiol comprising hydrolysis-decarboxylation without the use of alcohol. Such method provided in the present disclosure is environment-friendly, cost-effective and safe with good yield and quality.

The present disclosure provides a method for preparing cannabidiol represented by formula (I) below, 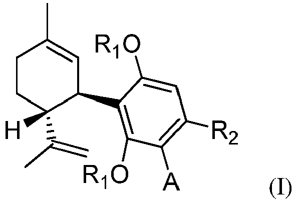 comprising hydrolysis-decarboxylation of an intermediate compound represented by formula (II) below, 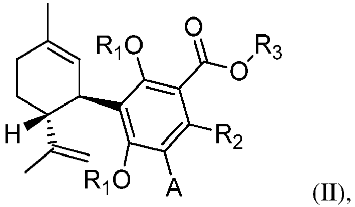 wherein R₁ is H, a C₁-C₁₆ alkyl group or a protecting group, R2 is an alkyl group or an ether group, R3 is a C₁-C₁₆ alkyl group or a protecting group, A is H or a COO-C₁-C₅ group, wherein C₁-C₅ is a straight or branched alkyl chain.

In an embodiment of the present disclosure, the protecting group may be a hydroxyl protecting group. In another embodiment, the hydroxyl protecting group is selected from the group consisting of tri-*i*-propylsilyloxymethyl (TOM), (phenyldimethylsilyl) methoxymethyl (SMOM), acetyl (Ac), pivaloyl (Piv), benzoate (Bz), trimethylsilyl (TMS), t-butyldimethylsilyl (TBDMS), triisopropylsilyl (TIPS), tri(trimethylsilyl)silyl (TTMSS), and t-butyldiphenylsilyl (TBDPS). In a further embodiment, the protecting group is represented by the formula O-SiRₓR_{y}R_{z}, wherein the substituents on the Si atom, namely Rₓ, R_{y} and R_{z}, are required in order to satisfy the bonding orbital of the Si atom. The use of any particular substituent Rₓ, R_{y} or R_{z} is not especially limited. In an embodiment of the present disclosure, each of Rₓ, R_{y} and R_{z} is a C₁ or C₂ hydrocarbyl group (i.e., methyl or ethyl) simply because such materials are readily synthesized from commercially available materials.

In an embodiment of the present disclosure, the method further comprises preparation of the intermediate compound represented by formula (II), comprising: Lewis acid or Brønsted acid-promoted condensation of esterified olivetol represented by formula (III) below, 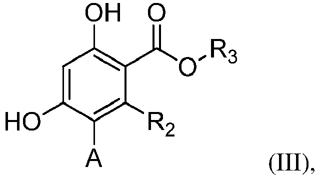 with terpene represented by formula (IV), 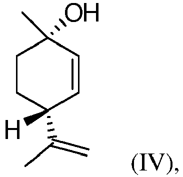 wherein R₂, R₃ and A are described as above.

In an embodiment of the present disclosure, the method further comprises extraction of the intermediate compound represented by formula (II) with a solvent after Lewis acid or Brønsted acid-promoted condensation of esterified olivetol represented by formula (III). In a further embodiment, the solvent is an organic solvent. In another embodiment, the solvent is aprotic. In still another embodiment, the solvent is one with a low boiling point. In an embodiment, the solvent is a C₅-C₇ hydrocarbon that boils above 30 °C and below 100 °C. In a further embodiment, the solvent is pentane, cyclopentane, hexane, cyclohexane, heptane, or a mixture thereof. In another embodiment, the solvent is hexane.

In an embodiment of the present disclosure, the method for preparing cannabidiol represented by formula (I) is illustrated by scheme 6 shown as follows:

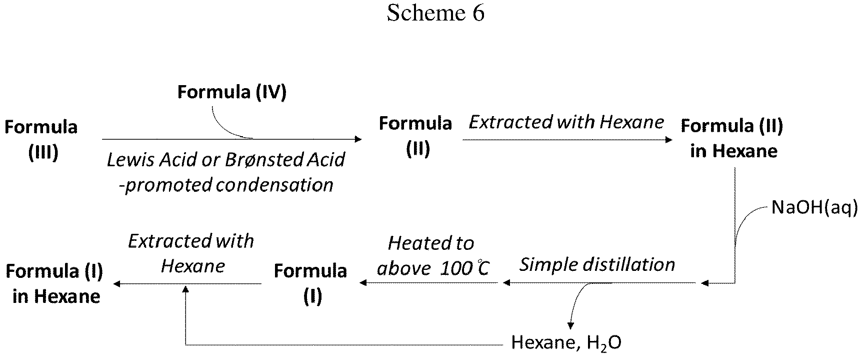

In the above scheme, simple distillation is carried out before the hydrolysis- decarboxylation of formula (II) to remove the organic solvent, hexane, and water to obtain neat formula (II). The distillate can be collected and reused for extraction of formula (I) prior to further purification. After distillation, the reaction is heated to above 100°C, and the neat formula (II) converts to formula (I) in a solvent-free state under atmospheric pressure.

In an embodiment of the present disclosure, formula (I) is prepared by hydrolysis-decarboxylation of formula (II) in absence of solvent. In a further embodiment of the present disclosure, formula (I) is prepared by hydrolysis-decarboxylation of formula (II) in an absence of alcohol. In an embodiment, the hydrolysis-decarboxylation of formula (II) is reacting in a neat state. In an embodiment, the hydrolysis-decarboxylation of formula (II) is reacting in a neat state by removing water and the solvent in the reaction. In a further embodiment, the water and solvent are removed from the reaction of hydrolysis-decarboxylation of formula (II) by simple distillation, such that the hydrolysis- decarboxylation of formula (II) is reacting in a neat state in absence of a solvent. In an embodiment, the hydrolysis-decarboxylation of formula (II) is reacting in a neat state in presence of base, e.g., a NaOH solution. In an embodiment, the hydrolysis-decarboxylation of formula (II) is carried out under atmospheric pressure.

In an embodiment of the present disclosure, the hydrolysis-decarboxylation of the intermediate compound represented by formula (II) is carried out above 100 °C. In an embodiment of the present disclosure, the temperature is in a range from 101 °C to 130 °C. In an embodiment of the present disclosure, the temperature is in a range from 105 °C to 125 °C. In another embodiment of the present disclosure, the temperature to carry out hydrolysis-decarboxylation of the intermediate compound represented by formula (II) is 101 °C, 102 °C, 103 °C, 104 °C, 105 °C, 106 °C, 107 °C, 108 °C, 109 °C, 110 °C, 111 °C, 112 °C, 113 °C, 114 °C, 115 °C, 116 °C, 117 °C, 118 °C, 119 °C, 120 °C, 121 °C, 122 °C, 123 °C, 124 °C, 125 °C, 126 °C, 127 °C, 128 °C, 129 °C, or 130 °C.

In an embodiment of the present disclosure, the hydrolysis-decarboxylation of the intermediate compound represented by formula (II) is carried out in the presence of a base. In an embodiment, the base is a hydroxide of alkali metal. In an embodiment of the present disclosure, the base is NaOH or KOH.

In an embodiment of the present disclosure, the equivalent of the base is 3 to 5.

In an embodiment of the present disclosure, the base is NaOH, and the equivalent of NaOH is 3 to 5. In another embodiment of the present disclosure, the base is KOH, and the equivalent of KOH is 3 to 5.

In an embodiment of the present disclosure, condensation of formula (III) with formula (IV) is promoted by Lewis acid or Brønsted acid in presence of dichloromethane (DCM). In an embodiment of the present disclosure, the Lewis acid is boron trifluoride etherate, aluminium chloride, indium chloride, trimethylsilyl trifluoromethanesulfonate, stannic chloride, zinc chloride, zinc trifluoromethanesulfonate, ferric chloride, ferrous chloride, titanium chloride, scandium trimethylsilyl trifluoromethanesulfonate, or lanthanum trifluoromethanesulfonate. In another embodiment of the present disclosure, the Brønsted acid is p-toluenesulfonic acid or trifluoroacetic acid.

In an embodiment of the present disclosure, the Lewis acid is boron trifluoride etherate (BF₃·OEt₂).

The present disclosure provides a method for industrial production of cannabidiol that overcomes the defects of previously existing methods, for example, (a) saving the cost of raw material, alcohol, which is not required in the reaction; (b) saving the cost of processing alcohol waste; (c) economical and environmentally friendly by reusing the distillate; and (d) carrying out the hydrolysis-decarboxylation in a neat reaction under atmospheric pressure with no safety concern of the high pressure caused by the solvent during heating. Therefore, the present disclosure provides a method for cannabidiol synthesis that is safe and economical, and can be readily adopted in industrial scale operation in preference over the previously existing methods.

The following are examples further demonstrating the efficacy of the present disclosure, but are not to limit the scope of the present disclosure.

### EXAMPLE

### Example 1: Preparation of Formula (I) via ethyl olivetolate (Formula III) with NaOH through hydrolysis-decarboxylation heated at 115 °C as a neat reaction

Ethyl olivetolate (Formula III) (252 g, 1 mole) was diluted in dichloromethane, and the mixture was cooled to 0 °C followed by addition of BF₃·Et₂O (28 g, 0.2 mole). Mixture of p-mentha-2,8-dien-1-ol (152 g, 1 mole) in dichloromethane was added into the ethyl olivetolate solution at 0 °C, and the reaction mixture was stirred for one hour at 0 °C before quenched by 10% Na₂CO₃. The organic layer was subsequently collected and concentrated after phase separation, and the residue was diluted in hexane followed by washing with water twice to afford the crude compound of Formula II (301.3 g, 78.1 wt.% assayed by ultra-performance liquid chromatography (UPLC)).

The compound of Formula II in hexane (total weight 600 g) was subjected to conversion into the compound of Formula I without further purification. The crude compound of Formula II in hexane (120 g, 0.2 mole) was mixed with 20% NaOH(aq) (160 g, 0.8 mole) in a stainless reactor at ambient temperature. Simple distillation was carried out to remove hexane and water in the reaction mixture, and then heated at 115 °C for 2 hours to afford the crude compound of Formula I (44.1 g, 70.3 wt.% with a 2-step reaction from ethyl olivetolate assayed by UPLC).

Yield of hydrolysis-decarboxylation was calculated as 90.1% for the reaction carried out in this example.

### Example 2: Preparation of Formula (I) via ethyl olivetolate (Formula III) with NaOH through hydrolysis-decarboxylation heated at 125 °C as a neat reaction

Ethyl olivetolate (Formula III) (252 g, 1 mole) was diluted in dichloromethane, and the mixture was cooled to 0 °C followed by addition of BF₃·Et₂O (28 g, 0.2 mole). Mixture of p-mentha-2,8-dien-1-ol (152 g, 1 mole) in dichloromethane was added into the ethyl olivetolate solution at 0 °C, and the reaction mixture was stirred for one hour at 0 °C before quenched by 10% Na₂CO₃. The organic layer was subsequently collected and concentrated after phase separation, and the residue was diluted in hexane followed by washing with water twice to afford the crude compound of Formula II (301.3 g, 78.1 wt.% assayed by UPLC). The compound of Formula II in hexane (total weight 600 g) was subjected to conversion into compound (I) without further purification.

The crude compound of Formula II in hexane (120 g, 0.2 mole) was mixed with 20% NaOH(aq) (160 g, 0.8 mole) in a stainless reactor at ambient temperature. Simple distillation was carried out to remove hexane and water in the reaction mixture, and then heated at 125 °C for 2 hours to afford the crude compound of Formula I (42.4 g, 67.6 wt.% with a 2-step reaction from ethyl olivetolate assayed by UPLC).

Yield of hydrolysis-decarboxylation was calculated as 86.6% for the reaction carried out in this example.

### Example 3: Preparation of Formula (I) via ethyl olivetolate (Formula III) with NaOH through hydrolysis-decarboxylation heated at 105 °C as a neat reaction

Ethyl olivetolate (Formula III) (252 g, 1 mole) was diluted in dichloromethane, and the mixture was cooled to 0 °C followed by addition of BF₃·Et₂O (28 g, 0.2 mole). Mixture of p-mentha-2,8-dien-1-ol (152 g, 1 mole) in dichloromethane was added into the ethyl olivetolate solution at 0 °C, and the reaction mixture was stirred for one hour at 0 °C before quenched by 10% Na₂CO₃. The organic layer was subsequently collected and concentrated after phase separation, and the residue was diluted in hexane followed by washing with water twice to afford the crude compound of Formula II (301.3 g, 78.1 wt.% assayed by UPLC). The compound of Formula II in hexane (total weight 600 g) was subjected to conversion into the compound of Formula I without further purification.

The crude compound of Formula II in hexane (120 g, 0.2 mole) was mixed with 20% NaOH(aq) (160 g, 0.8 mole) in a stainless reactor at ambient temperature. Simple distillation was carried out to remove hexane and water in the reaction mixture, and then heated at 105 °C for 2 hours to afford the crude compound of Formula I (43.2 g, 68.8 wt.% with a 2-step reaction from ethyl olivetolate assayed by UPLC).

Yield of hydrolysis-decarboxylation was calculated as 88.2% for the reaction carried out in this example.

### Example 4: Preparation of Formula (I) via ethyl olivetolate (Formula III) with KOH through hydrolysis-decarboxylation heated at 115 °C as a neat reaction

Ethyl olivetolate (Formula III) (252 g, 1 mole) was diluted in dichloromethane, and the mixture was cooled to 0 °C followed by addition of BF₃·Et₂O (28 g, 0.2 mole). Mixture of p-mentha-2,8-dien-1-ol (152 g, 1 mole) in dichloromethane was added into the ethyl olivetolate solution at 0 °C, and the reaction mixture was stirred for one hour at 0 °C before quenched by 10% Na₂CO₃. The organic layer was subsequently collected and concentrated after phase separation, and the residue was diluted in hexane followed by washing with water twice to afford the crude compound of Formula II (301.3 g, 78.1 wt.% assayed by UPLC). The compound of Formula II in hexane (total weight 600 g) was subjected to conversion into the compound of Formula I without further purification. The crude compound of Formula II in hexane (120 g, 0.2 mole) was mixed with 20% KOH(aq) (209 g, 0.8 mole) in a stainless reactor at ambient temperature. Simple distillation was carried out to remove hexane and water in the reaction mixture, and then heated at 115 °C for 1 hour to afford the crude compound of Formula I (42.0 g, 71.2 wt.% with a 2-step reaction from ethyl olivetolate assayed by UPLC).

Yield of hydrolysis-decarboxylation was calculated as 91.2% for the reaction carried out in this example.

Example 5: Preparation of Formula (I) via methyl olivetolate (Formula III) with NaOH through hydrolysis-decarboxylation heated at 115 °C as a neat reaction Methyl olivetolate (Formula III) (47.6 g, 0.2 mole) was diluted in dichloromethane, and the mixture was cooled to 0 °C followed by addition of BF₃·Et₂O (6 g, 0.04 mole). Mixture of p-mentha-2,8-dien-1-ol (30.4 g, 0.2 mole) in dichloromethane was added into the methyl olivetolate solution at 0 °C, and the reaction mixture was stirred for one hour at 0 °C before quenched by 10% Na₂CO₃. The organic layer was subsequently collected and concentrated after phase separation, and the residue was diluted in hexane followed by washing with water twice to afford the crude compound of Formula II (63.6 g, 85 wt.% assayed by UPLC). The compound of Formula II in hexane (total weight 208 g) was subjected to conversion to the compound of Formula I without further purification.

The crude compound of Formula II in hexane (63.6 g, 0.2 mole) was mixed with 20% NaOH(aq) (160 g, 0.8 mole) in a stainless reactor at ambient temperature. Simple distillation was carried out to remove hexane and water in the reaction mixture, and then heat at 115 °C for 1 hour to afford the crude compound of Formula I (39.8 g, 63.8 wt.% with a 2-step reaction from methyl olivetolate assayed by UPLC).

Yield of hydrolysis-decarboxylation was calculated as 75.1% for the reaction carried out in this example.

## Claims

1. A method for preparing cannabidiol represented by Formula (I), 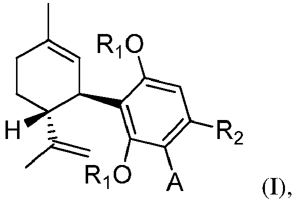
comprising hydrolysis-decarboxylation of a compound represented by Formula (II) in a solvent-free state at atmospheric pressure, 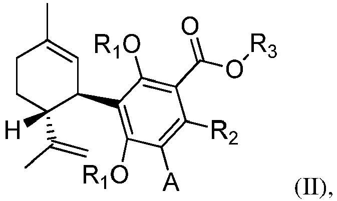
wherein R₁ is H, an alkyl group or a protecting group, R2 is an alkyl group or an ether group, R3 is an alkyl group or a protecting group, and A is H or a carboxylic ester group
wherein the compound represented by Formula (II) is prepared by Lewis acid or Brønsted acid-promoted condensation of a compound represented by Formula (III), 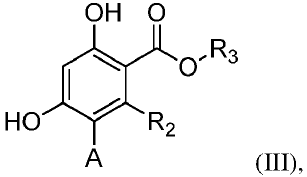
with a compound represented by Formula (IV),

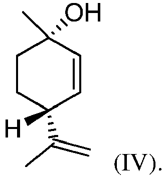

2. The method of claim **1**, further comprising removing water and a solvent for the hydrolysis-decarboxylation to be carried out in the solvent-free state.

3. The method of claim **2**, further comprising performing distillation to remove water and the solvent in the hydrolysis-decarboxylation.

4. The method of claim **2**, wherein the solvent to be removed is C₅-C₇ hydrocarbon with a boiling point above 30 °C and below 100 °C.

5. The method of claim **4**, wherein the solvent is hexane.

6. The method of claim **1**, further comprising heating at a temperature in a range from 101 °C to 130 °C in the hydrolysis-decarboxylation.

7. The method of claim **1**, wherein the hydrolysis-decarboxylation is performed in a presence of a base, preferably a hydroxide of an alkali metal, preferably KOH or NaOH.

8. The method of claim **7**, wherein the base has an equivalent of from 3 to 5.

9. The method of claim **1**, wherein the Lewis acid is selected from the group consisting of boron trifluoride etherate, aluminium chloride, indium chloride, trimethylsilyl trifluoromethanesulfonate, stannic chloride, zinc chloride, zinc trifluoromethanesulfonate, ferric chloride, ferrous chloride, titanium chloride, scandium trimethylsilyl trifluoromethanesulfonate, and lanthanum trifluoromethanesulfonate, preferably boron trifluoride etherate.

10. The method of claim **1**, wherein the Brønsted acid- acid is p-toluenesulfonic acid or trifluoroacetic acid

11. The method of claim **10**, wherein R₂ is a C₅ alkyl group, R3 is a C₁-C₂ alkyl group and A is H in the compound represented by Formula (III).

12. The method of claim **1**, wherein R₁ is H, R₂ is a C₅ alkyl group and A is H in the compound represented by Formula (I).

13. The method of claim **1**, wherein R₁ is H, R₂ is a C₅ alkyl group, R3 is a C₁-C₂ alkyl group and A is H in the compound represented by Formula (II).

14. The method of claim **1**, wherein the protecting group is selected from the group consisting of tri-i-propylsilyloxymethyl (TOM), (phenyldimethylsilyl)methoxymethyl (SMOM), acetyl (Ac), pivaloyl (Piv), benzoate (Bz), trimethylsilyl (TMS), t-butyldimethylsilyl (TBDMS), triisopropylsilyl (TIPS), tri(trimethylsilyl)silyl (TTMSS), and t-butyldiphenylsilyl (TBDPS).

## Patentansprüche

1. Verfahren zur Herstellung von Cannabidiol, dargestellt durch Formel (I), 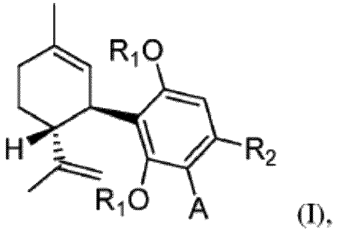
umfassend eine Hydrolyse-Decarboxylierung einer Verbindung, dargestellt durch Formel (II), in einem lösungsmittelfreien Zustand bei atmosphärischem Druck, 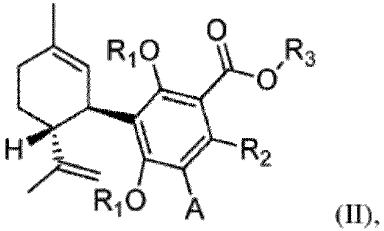
wobei R₁ H, eine Alkylgruppe oder eine Schutzgruppe ist, R2 eine Alkylgruppe oder eine Ethergruppe ist, R₃ eine Alkylgruppe oder eine Schutzgruppe ist und A H oder eine Carbonsäureestergruppe ist,
wobei die Verbindung, dargestellt durch Formel (II), durch eine mit einer Lewis-Säure oder einer Brønsted-Säure geförderte Kondensation einer Verbindung, dargestellt durch Formel (III), 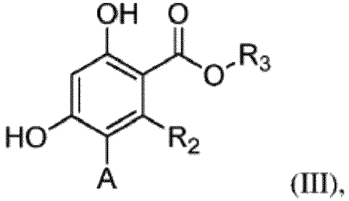
mit einer Verbindung, dargestellt durch Formel (IV), 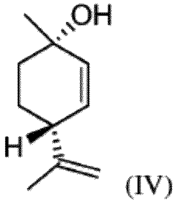
hergestellt wird.

2. Verfahren nach Anspruch **1**, weiterhin umfassend ein Abziehen von Wasser und einem Lösungsmittel, damit die Hydrolyse-Decarboxylierung in dem lösungsmittelfreien Zustand ausgeführt wird.

3. Verfahren nach Anspruch **2**, weiterhin umfassend ein Durchführen einer Destillation, um Wasser und das Lösungsmittel abzuziehen, in der Hydrolyse-Decarboxylierung.

4. Verfahren nach Anspruch **2**, wobei das abzuziehende Lösungsmittel C₅-C₇-Kohlenwasserstoff mit einem Siedepunkt über 30 °C und unter 100 °C ist.

5. Verfahren nach Anspruch **4**, wobei das Lösungsmittel Hexan ist.

6. Verfahren nach Anspruch **1**, weiterhin umfassend ein Erhitzen bei einer Temperatur in einem Bereich von 101 °C bis 130 °C in der Hydrolyse-Decarboxylierung.

7. Verfahren nach Anspruch **1**, wobei die Hydrolyse-Decarboxylierung in Gegenwart einer Base, vorzugsweise eines Hydroxids eines Alkalimetalls, vorzugsweise KOH oder NaOH, durchgeführt wird.

8. Verfahren nach Anspruch **7**, wobei die Base ein Äquivalent von 3 bis 5 aufweist.

9. Verfahren nach Anspruch **1**, wobei die Lewis-Säure aus der Gruppe bestehend aus Bortrifluoridetherat, Aluminiumchlorid, Indiumchlorid, Trimethylsilyltrifluormethansulfonat, Zinnchlorid, Zinkchlorid, Zinktrifluormethansulfonat, Eisen(III)-chlorid, Eisen(II)-chlorid, Titanchlorid, Scandiumtrimethylsilyltrifluormethansulfonat und Lanthantrifluormethansulfonat ausgewählt ist, vorzugsweise Bortrifluoridetherat ist.

10. Verfahren nach Anspruch **1**, wobei die Brønsted-Säure p-Toluolsulfonsäure oder Trifluoressigsäure ist.

11. Verfahren nach Anspruch **10**, wobei in der Verbindung, dargestellt durch Formel (III), R2 eine C₅-Alkylgruppe ist, R3 eine C₁-C₂-Alkylgruppe ist und A H ist.

12. Verfahren nach Anspruch **1**, wobei in der Verbindung, dargestellt durch Formel (I), R₁ H ist, R₂ eine C₅-Alkylgruppe ist und A H ist.

13. Verfahren nach Anspruch **1**, wobei in der Verbindung, dargestellt durch Formel (II), R₁ H ist, R₂ eine C₅-Alkylgruppe ist, R3 eine C₁-C₂-Alkylgruppe ist und A H ist.

14. Verfahren nach Anspruch **1**, wobei die Schutzgruppe aus der Gruppe bestehend aus Tri-i-propylsilyloxymethyl (TOM), (Phenyldimethylsilyl)methoxymethyl (SMOM), Acetyl (Ac), Pivaloyl (Piv), Benzoat (Bz), Trimethylsilyl (TMS), t-Butyldimethylsilyl (TBDMS), Triisopropylsilyl (TIPS), Tri(trimethylsilyl)silyl (TTMSS) und t-Butyldiphenylsilyl (TBDPS) ausgewählt ist.

## Revendications

1. Procédé de préparation de cannabidiol représenté par la Formule (I), 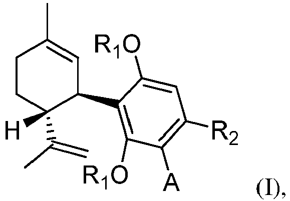
comprenant une hydrolyse-décarboxylation d'un composé représenté par la Formule (II) dans un état sans solvant à pression atmosphérique, 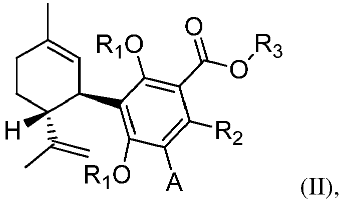
dans lequel R₁ est H, un groupe alkyle ou un groupe protecteur, R₂ est un groupe alkyle ou un groupe éther, R₃ est un groupe alkyle ou un groupe protecteur, et A est H ou un groupe ester carboxylique
dans lequel le composé représenté par la Formule (II) est préparé par condensation promue par un acide de Lewis ou un acide de Brønsted d'un composé représenté par la Formule (III), 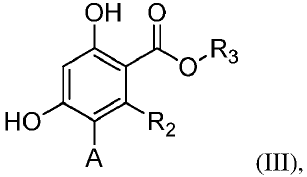
avec un composé représenté par la Formule (IV),

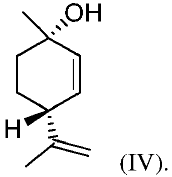

2. Procédé selon la revendication **1**, comprenant en outre l'élimination d'eau et d'un solvant pour que l'hydrolyse-décarboxylation soit effectuée dans l'état sans solvant.

3. Procédé selon la revendication **2**, comprenant en outre la réalisation d'une distillation pour éliminer l'eau et le solvant dans l'hydrolyse-décarboxylation.

4. Procédé selon la revendication **2**, dans lequel le solvant à éliminer est un hydrocarbure en C₅-C₇ avec un point d'ébullition au-dessus de 30 °C et en-dessous de 100 °C.

5. Procédé selon la revendication **4**, dans lequel le solvant est l'hexane.

6. Procédé selon la revendication **1**, comprenant en outre le chauffage à une température dans une plage de 101 °C à 130 °C dans l'hydrolyse-décarboxylation.

7. Procédé selon la revendication **1**, dans lequel l'hydrolyse-décarboxylation est réalisée en présence d'une base, de préférence un hydroxyde d'un métal alcalin, de préférence KOH ou NaOH.

8. Procédé selon la revendication **7**, dans lequel la base a un équivalent de 3 à 5.

9. Procédé selon la revendication **1**, dans lequel l'acide de Lewis est sélectionné dans le groupe constitué de diéthérate de trifluorure de bore, chlorure d'aluminium, chlorure d'indium, trifluorométhylsulfonate de triméthylsilyle, chlorure stannique, chlorure de zinc, trifluorométhylsulfonate de zinc, chlorure de fer(III), chlorure de fer(II), chlorure de titanium, trifluorométhylsulfonate triméthylsilyle de scandium, et trifluorométhylsulfonate de lanthane, de préférence le diéthérate de trifluorure de bore.

10. Procédé selon la revendication **1**, dans lequel l'acide de Brønsted est l'acide p-toluènesulfonique ou l'acide trifluoroacétique.

11. Procédé selon la revendication **10**, dans lequel R2 est un groupe alkyle en C₅, R₃ est un groupe alkyle en C₁-C₂ et A est H dans le composé représenté par la Formule (III).

12. Procédé selon la revendication **1**, dans lequel R₁ est H, R₂ est un groupe alkyle en C₅ et A est H dans le composé représenté par la Formule (I).

13. Procédé selon la revendication **1**, dans lequel R₁ est H, R₂ est un groupe alkyle en C₅, R₃ est un groupe alkyle en C₁-C₂ et A est H dans le composé représenté par la Formule (II).

14. Procédé selon la revendication **1**, dans lequel le groupe protecteur est sélectionné dans le groupe constitué de tri-i-propylsilyloxyméthyle (TOM), (phenyldiméthylsilyl)méthoxyméthyle (SMOM), acétyle (Ac), pivaloyle (Piv), benzoate (Bz), triméthylsilyle (TMS), t-butyldiméthylsilyle (TBDMS), triisopropylsilyle (TIPS), tri(triméthylsilyl)silyle (TTMSS), et t-butyldiphénylsilyle (TBDPS).
